# EUROPÄISCHE PATENTSCHRIFT

(11) **EP 0 563 503 B1**
(45) Veröffentlichungstag und Bekanntmachung des Hinweises auf die Patenterteilung: **27.12.1996**
(21) Anmeldenummer: 92810254.0
(22) Anmeldetag: 03.04.1992
(51) Int. Cl.: A61F 2/34

(54) **Aussenschale für eine künstliche Hüftgelenkspfanne**
Outer shell for an artificial acetabular cup
Coquille extérieure pour une cupule acétabulaire artificielle

(43) Veröffentlichungstag der Anmeldung: 06.10.1993
(73) Patentinhaber: Sulzer Orthopädie AG, 6340 Baar (CH)
(72) Erfinder: Wagner, Heinz, Prof. Dr., Orthopädische Klinik, W-8501 Schwarzenbruck (DE); Roland, Willi, CH-8413 Neftenbach (CH)
(74) Vertreter: Trieblnig, Adolf

(56) Entgegenhaltungen:
- EP-A- 0 242 719
- DE-A- 3 027 063
- DE-B- 2 410 057
- DE-U- 8 808 699

## Beschreibung

Die Erfindung betrifft eine metallische Aussenschale für eine künstliche Hüftgelenkspfanne, die zur Befestigung im Beckenknochen an ihrem äquatorialen Rand mit Stützlappen versehen ist, die ihrerseits Bohrungen für den Durchtritt von Knochenschrauben aufweisen.

Aussenschalen der genannten Art sind beispielsweise bekannt aus der EP-A-0242719 oder aus der FR-A-2595241 (8603853); die Stützlappen derartige Aussenschalen müssen häufig, besonders bei Reoperationen intraoperativ an den Beckenknochen angepasst werden, der gerade bei Reoperationen oft beschädigt oder stark degeneriert ist. Der Operateur hat bei den genannten, bekannten Konstruktionen vielfach Schwierigkeiten, die Stützlappen in die richtige Form Zu bringen. Der Erfindung liegt daher die Aufgabe zugrunde, die Arbeit des Operateurs in den genannten Fällen zu erleichtern und eine exakte Anpassung der Stützlappen an den Knochen zu erleichtern; weiterhin soll durch Biegen entlang der vorgegebenen Sollbiegelinien vermieden werden, dass die Festigkeit der Stützlappen durch unkontrolliertes Biegen, beispielsweise durch Durchtrittsbohrungen für die Knochenschrauben, unzulässig verringert wird.

Diese Aufgabe wird mit der vorliegenden Erfindung dadurch gelöst, dass die Stützlappen mit rillenartigen Sollbiegelinien versehen sind. Entlang diesen Linien besteht dadurch ein verminderter Widerstand gegen plastische Verformungen, so dass sich die Lappen vorzugsweise entlang den Linien verformen.

Um eine unzulässige Schwächung der Stützlappen zu vermeiden, ist es vorteilhaft, wenn die Biegelinien ausschliesslich im durch die Bohrungen ungestörten Bereich der Stützlappenflächen verlaufen. Weiterhin hat es sich als zweckmässig erwiesen, wenn die rillenartigen Biegelinien auf der äusseren d.h. dem Knochen abgewandten Oberfläche der Lappen angeordnet sind, da dann ihr Verlauf dem Operateur beim probeweisen Einsetzen und Anpassen der Aussenschale sichtbar ist. Um ein Abbiegen in Bereichen sich schneidender Biegelinien zu erleichtern, können an deren Schnittstellen runde Vertiefungen vorgesehen sein.

Im folgenden wird die Erfindung anhand von Ausführungsbeispielen im Zusammenhang mit der Zeichnung näher erläutert.
- Fig.1: zeigt in einem Schnitt I - I von Fig.2 ein Ausführungsbeispiel der neuen Aussenschale;
- Fig.2: zeigt in schematischer Darstellung die Aufsicht in Richtung der Polachse auf den "Äquator" einer erfindungsgemässen Aussenschale und
- Fig.3: schliesslich gibt in einem Schnitt IV - IV einen vergrösserten Schnitt quer zu einer Biegelinie aus Fig.2 wieder.

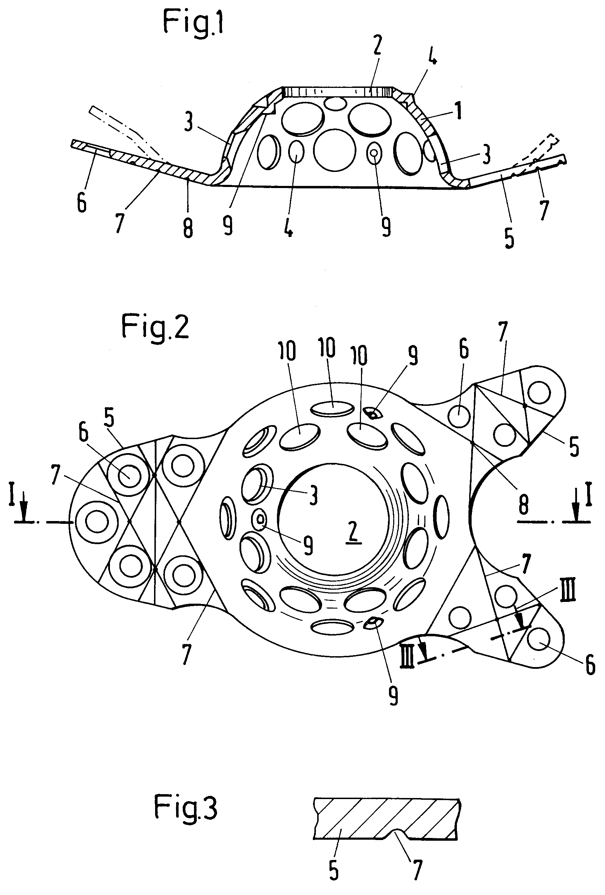

Die beispielsweise aus Titan bestehende Stütz- oder Aussenschale 1 (Fig. 1) für eine Verankerung mit Knochenzement ist in ihrer Grundform eine Halbkugel, in deren Polbereich eine Kalotte abgeschnitten ist, so dass die Schale 1 dort eine Öffnung 2 aufweist. In der Schalenfläche sind angesenkte Durchtrittsöffnungen 3 für nicht gezeigte Knochenschrauben vorgesehen, mit deren Hilfe die Schale 1 im Beckenknochen fixiert werden kann. Ausserdem weist die Schalenfläche Kontrollöffnungen 10 in Form von Bohrungen auf, durch die der Abstand zum dahinter liegenden Knochengewebe kontrolliert werden kann und durch die Zement in den Zwischenraum zum Knochengewebe eingespritzt werden kann.

Weiterhin sind auf der Schalenfläche aussen Noppen 4 verteilt, die beim Einschlagen der Aussenschale diese am Knochenbett vorfixieren. Ebenso sind auf der Innenseite der Aussenschale drei Noppen 9 angebracht, die eine einzementierbare Innenschale so auf Distanz halten, dass zwischen den beiden Schalen ein Zementbett konstanter Dicke erzeugt wird, in dem die Noppenhöhe dem zu erzeugenden "Spalt" entspricht.

Entlang dem äquatorialen Rand der Schale 1 sind zusätzlich Stützlappen oder Laschen 5 vorgesehen, die ebenfalls mit Durchtrittsbohrungen 6 für Knochenschrauben versehen sind. Diese Laschen 5 haben die Aufgabe die Schale 1 zusätzlich am Becken zu fixieren. Da sie dafür durch Biegen an den Knochen angepasst werden müssen, sind sie plastisch verformbar.

Um ein Biegen in vorgegebenen Richtungen zu gewährleisten, sind die Stützlappen 5 von Sollbiegelinien 7 durchzogen, die als rillenartige Vertiefungen verwirklicht sind; sie befinden sich vorzugsweise auf der Aussenseite, d. h. auf der dem Knochen abgewandten Seite der Laschen 5. Weiterhin sind die Biegelinien 7 so in die Laschen gelegt, dass sie keine der Durchtrittsbohrungen 6 schneiden. Dadurch wird eine unzulässige Schwächung der Laschenfestigkeit vermieden und es wird verhindert, dass sich die deformierten Laschen bei der Fixierung mit Knochenschrauben zurückbiegen.

An den Schnittstellen von zwei oder mehr Biegelinien 7 ist jeweils eine runde Vertiefung 8 vorgesehen, durch die ein mehrfaches Biegen entlang sich kreuzenden Biegelinien 7 - das ja im allgemeinen durch den Operateur während der Operation erfolgt - erleichtert wird.

Sowohl die Biegelinien 7 als auch die Vertiefungen 8 werden beispielsweise mit Hilfe von Prägestempeln in die Oberfläche der Stützlappen 5 eingepresst.

## Patentansprüche

1. Metallische Aussenschale (1) für eine künstliche Hüftgelenkspfanne, die zur Befestigung am Beckenknochen an ihrem äquatorialen Rand mit Stützlappen (5) versehen ist, die ihrerseits Bohrungen (6) für den Durchtritt von Knochenschrauben und als Kontrollöffnungen für den Knochenabstand aufweisen, dadurch gekennzeichnet, dass die Stützlappen (5) mit rillenartigen Sollbiegelinien (7) versehen sind.

2. Aussenschale nach Anspruch 1, dadurch gekennzeichnet, dass die Biegelinien (7) ausschliesslich im, durch die Bohrungen (6), ungestörten Bereich der Stützlappenflächen verlaufen.

3. Aussenschale nach Anspruch 1 oder 2, dadurch gekennzeichnet, dass die rillenartigen Biegelinien (7) auf der äusseren Oberfläche der Lappen (5) angeordnet sind.

4. Aussenschale nach einem der Ansprüche 1 bis 3, dadurch gekennzeichnet, dass in den Schnittstellen von Biegelinien (7) runde Vertiefungen (8) vorgesehen sind.

## Claims

1. A metallic outer cup (1) for an artificial hipjoint socket, which for fastening to the pelvic bone is provided at its equatorial edge with supporting flaps (5) which in turn exhibit holes (6) drilled for bone screws to pass through and as openings for checking the clearance from the bone, characterized in that the supporting flaps (5) are provided with preset lines of bend (7) like grooves.

2. An outer cup as in Claim 1, characterized in that the lines of bend (7) run exclusively in the region of the supporting flaps undisturbed by the drilled holes (6).

3. An outer cup as in Claim 1 or 2, characterized in that the groovelike lines of bend (7) are arranged on the outer surface of the flaps (5).

4. An outer cup as in one of the Claims 1 to 3, characterized in that round depressions are provided at the intersections between the lines of bend (7).

## Revendications

1. Coquille extérieure (1) métallique pour une cupule acétabulaire artificielle, qui, pour assurer la fixation sur l'os du bassin, est pourvue, sur son bord équatorial, de languettes d'appui (5), présentant de leur côté des perçages (6) destinés au passage de vis à os et d'ouvertures de contrôle destinées à vérifier l'espacement vis à vis de l'os, caractérisée en ce que les languettes d'appui (5) sont pourvues de lignes destinées à la flexion (7), se présentant sous forme de cannelures.

2. Coquille extérieure selon la revendication 1, caractérisée en ce que les lignes de flexion (7) s'étendent exclusivement dans la zone, non perturbée par les perçages (6), des surfaces des languettes d'appui.

3. Coquille extérieure selon la revendication 1 ou 2, caractérisée en ce que les lignes de flexion (7) en forme de cannelures sont disposées sur la surface extérieure des languettes (5).

4. Coquille extérieure selon l'une des revendications 1 à 3, caractérisée en ce que des creusements (8) ronds sont prévus aux points d'intersection des lignes de flexion (7).
